(19) Europäisches Patentamt
European Patent Office
Office européen des brevets

(11) Numéro de publication : 0 373 213 B1

(12) # FASCICULE DE BREVET EUROPEEN

(45) Date de publication du fascicule du brevet : **21.10.92 Bulletin 92/43**

(51) Int. Cl.$^5$ : **A61K 31/505**

(21) Numéro de dépôt : **89906795.3**

(22) Date de dépôt : **29.05.89**

(86) Numéro de dépôt international : **PCT/FR89/00257**

(87) Numéro de publication internationale : **WO 89/11280 30.11.89 Gazette 89/28**

(54) **UTILISATION D'UNE SUBSTANCE FOLINIQUE EN TANT QU'AGENT ANTIAGREGANT PLAQUETTAIRE.**

(30) Priorité : **27.05.88 FR 8807075**

(43) Date de publication de la demande : **20.06.90 Bulletin 90/25**

(45) Mention de la délivrance du brevet : **21.10.92 Bulletin 92/43**

(84) Etats contractants désignés : **AT BE CH DE FR GB IT LI LU NL SE**

(56) Documents cités :
**ONKOLOGIE, vol. 7, no. 4, August 1984; R. STÄDTLER et al., pp. 238-242**
**SCAND. J. HAEMAT., vol. 10, 1973, Munksgaard, Copenhagen (DK); M.T.SHAW, pp. 24-27**
**BRITISH JOURNAL OF HAEMATOLOGY, vol. 32, 1976; B.A.COOPER et al., pp. 387-394**

(56) Documents cités :
**CLINICAL & EXPERIMENTAL PHARMACOLOGY & PHYSIOLOGY, supplement 5, 1979, Blackwell Scientific Publications; P.F.NIXON,pp. 35-41**
**THE MERCK INDEX, 1989, 11th ed.; pp. 660, 944-945**

(73) Titulaire : **SPIRAL RECHERCHE ET DEVELOPPEMENT**
**3, rue des Mardors Z.A. Couternon B.P. 1408**
**F-21560 Dijon Cédex (FR)**

(72) Inventeur : **PROST, Michel**
**15, route de Quétigny**
**F-21560 Couternon (FR)**

(74) Mandataire : **Clisci, Serge**
**S.A. FEDIT-LORIOT CONSEILS EN PROPRIETE INDUSTRIELLE 38, avenue Hoche**
**F-75008 Paris (FR)**

EP 0 373 213 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

### DOMAINE DE L'INVENTION

La présente invention concerne une nouvelle utilisation en thérapeutique de composés foliniques, à savoir l'acide 5-méthyl-5,6,7,8-tétrahydrofolique et ses sels, en tant que moyens antiagrégants plaquettaires.

### ART ANTERIEUR

On sait que l'acide folique, qui répond à la nomenclature systématique d'acide N-[4-[[(2-amino-1,4-dihydro-4-oxo-6-ptéridinyl)méthyl]amino]benzoyl]-L-glutamique, ou d'acide N-[p-[[(2-amino-4-hydroxy-6-ptéridinyl)méthyl]amino]benzoyl]-L-glutamic, et ses sels de métaux alcalins et alcalino-terreux agissent dans l'organisme en tant que substances vitaminiques [voir Merck Index, 10è édition (1983), pages 602-603].

On sait que l'acide folinique qui répond à la nomenclature systématique d'acide N-[4-[[(2-amino-5-formyl-1,4,5,6,7,8-hexahydro-4-oxo-6-ptéridinyl)méthyl]amino]benzoyl]-L-glutamique ou d'acide N-[p-[[(2-amino-5-formyl-5,6,7,8-tétrahydro-4-hydroxy-6-ptéridinyl)méthyl]amino]benzoyl]-L-glutamique, et à la nomenclature abrégée d'acide 5-formyl-5,6,7,8-tétrahydrofolique (ou 5-N-CHO-THF), et ses sels interviennent dans l'organisme en tant que substances antidotes vis-à-vis des antagonistes de l'acide folique [voir Merk Index, 10è édition (1983), page 603].

On sait que le méthotréxate, qui répond à la nomenclature systématique d'acide N-[4-[[(2,4-diamino-6-ptéridinyl)méthyl]méthylamino]benzoyl]-L-glutamique, et à la nomenclature abrégée d'acide 4-amino-$N^{10}$-méthylfolique ou MTX, est un antagoniste de l'acide folique et agit dans l'organisme en tant qu'agent antinéoplasique et antimétabolite. Plus précisément, on sait que le méthotréxate bloque (i) la conversion de l'acide folique en acide folinique et (ii) la synthèse de l'ADN, d'une part, et intervient en tant que substance induisant l'agrégation des plaquettes sanguines, d'autre part. On sait aussi que le méthotréxate a provoqué des accidents quand il a été administré à des patients atteint d'un cancer [voir Merk Index, 10è édition (1983), pages 859-860].

On sait en particulier que l'article de M.T. SHAW, Scand. J. Haemat., 10, pages 24-27, 1973, fait état, chez la femme enceinte présentant des tumeurs trophoblastiques, d'une diminution du nombre de plaquettes après administration d'un traitement comprenant en combinaison du méthotréxate (par voie I.V. ou I.M.) et de l'acide folinique (par voie I.M.). Le protocole suivi et les résultats fournis par ledit article ne permettent pas de suggérer que l'acide folinique a des propriétés antiagrégantes vis-à-vis des plaquettes sanguines, dès lors que notamment ledit acide n'a pas été utilisé seul. Il se trouve que l'acide folinique, selon les essais présentés ci-après, a des effets antiagrégants plaquettaires qui sont plus faibles que ceux de l'acide 5-méthyl-5,6,7,8-tétrahydrofolique.

Selon l'article de R. STADTLER et al., Onkologie, 7 (No 4), pages 228-242, (1984), l'assosiation MTX/5-N-CHO-THF inhiberait l'agrégation des plaquettes in vitro et n'agirait pas sur la numération des plaquettes ex vivo.

On sait que la méthoptérine, qui répond à la nomenclature systématique d'acide N-[4-[[(2-amino-1,4-dihydro-4-oxo-6-ptéridinyl )méthyl]méthylamino]-benzoyl]-4-L-glutamique ou d'acide N-[4-[[(2-amino-4-hydroxy-6-ptéridinyl)-méthyl]méthylamino]benzoyl]-L-glutamique, et qui est également dénommée acide $N^{10}$-méthylfolique, est un dérivé de l'acide folique qui a été décrit dans US-A-2 563 707 [voir également Merk Index, 10è édition (1983), page 859].

On sait enfin que l'acide 5-méthyl-5,6,7,8-tétrahydrofolique, qui répond à la nomenclature systématique d'acide N-[4-[[(2-amino-1,4,5,6,7,8-hexahydro-5-méthyl-4-oxo-6-ptéridinyl)méthyl]amino]benzoyl]-L-glutamique ou d'acide N-[4-[[(2-amino-5,6,7,8-tétrahydro-4-hydroxy-5-méthyl-6-ptéridinyl)méthyl] amino]benzoyl]-L-glutamique et a pour nomenclature abrégée 5-N-Me-THF, d'une part, et l'acide folinique, d'autre part, ont été proposés en thérapeutique en tant que médicaments anti-stress.

### OBJET DE L'INVENTION

Suivant l'invention on propose une nouvelle utilisation de substances foliniques en tant que moyens antiagrégants plaquettaires.

Plus précisément on préconise suivant l'invention une nouvelle utilisation en thérapeutique humaine ou vétérinaire d'une substance folinique, ladite utilisation étant caractérisée en ce que l'on fait appel à une substance choisie parmi l'ensemble constitué par (i) l'acide 5-méthyl-5,6,7,8-tétrahydrofolique,

(ii) ses sels avec les métaux alcalins et alcalino-terreux, et (iii) leurs mélanges, pour l'obtention d'un médicament antiagrégant plaquettaire destiné à une indication thérapeutique dans les cas où il convient d'inhiber ou réduire chez le mammifère l'agrégation des plaquettes sanguines.

Cette nouvelle utilisation en thérapeutique suivant l'invention n'est pas décrite ni suggérée par l'enseignement de l'art antérieur résumé ci-dessus.

DESCRIPTION DETAILLEE DE L'INVENTION

L'invention vise donc une nouvelle utilisation en tant que substances antiagrégantes plaquettaires de l'acide 5-méthyl-5,6,7,8-tetrahydrofolique et de ses sels.

Selon l'invention, la substance antiagrégante plaquettaire est choisie parmi l'ensemble comprenant

(i) l'acide 5-méthyl-5,6,7,8-tétrahydrofolique de formule

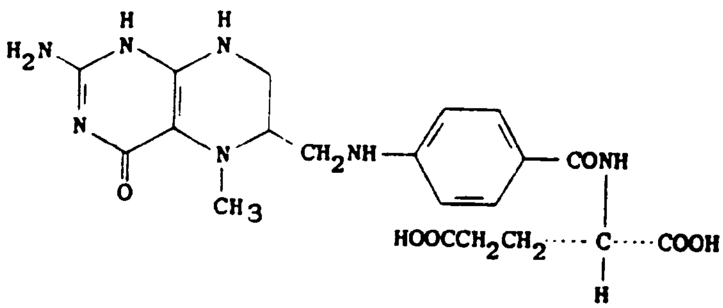

(I)

(ii) ses sels avec les métaux alcalins et alcalino-terreux; et,

(iii) leurs mélanges.

La formule I ci-dessus est parfois écrite sous la forme tautomère résultant de l'énolisation du groupe 4-oxo :

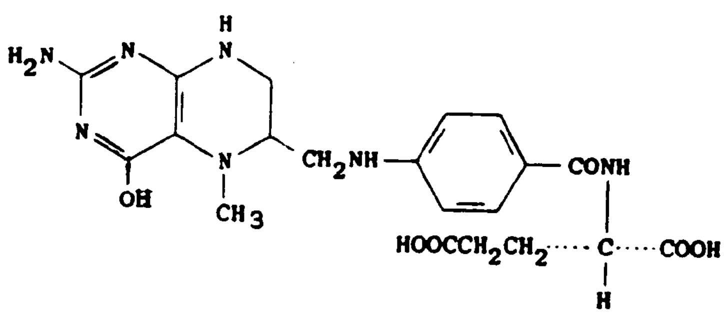

comme indiqué à la page 36 de l'article de P.F. NIXON "Folinic acid : pharmacokinetics and pharmacodynamics" dans Clinical and Experimental Pharmacology & Physiology, Suppl. 5, pages 35-41 (1979).

Les produits les plus intéressants suivant l'invention eu égard à leurs propriétés antiagrégantes plaquettaires sont surtout l'acide 5-méthyl-5,6,7,8-tétrahydrofolique et ses sels de sodium, potassium et calcium.

D'une façon pratique, on présume que l'efficacité de l'acide 5-méthyl-5,6,7,8-tétrahydrofolique est en relation avec le caractère hydrophobe du groupe 5-méthyle qui facilite ou améliore la biodisponibilité au niveau cellulaire, bien que le demandeur ne soit pas lié par cette théorie. Selon l'invention, ledit acide 5-méthyl-5,6,7,8-tétrahydrofolique et ses sels exercent, de manière générale, une activité antiagrégante plaquettaire supérieure à celle de l'acide folinique eu égard à leur pouvoir de pénétration dans les cellules.

On préconise suivant l'invention une composition thérapeutique qui renferme en association avec un excipient physiologiquement acceptable une quantité pharmaceutiquement efficace d'une substance antiagrégante plaquettaire appartenant à l'ensemble constitué par l'acide 5-méthyl-5,6,7,8-tétrahydrofolique, ses sels avec les métaux alcalins et alcalino-terreux, et leurs mélanges.

Une telle composition est utile en thérapeutique humaine et vétérinaire pour les animaux à sang chaud. Elle convient plus précisément pour le traitement des mammifères et surtout de l'homme.

Les principes actifs antiagrégants plaquettaires suivant l'invention peuvent être préparés selon une méthode connue en soi par application de mécanismes réactionnels classiques.

D'autres avantages et caractéristiques de l'invention seront mieux compris à la lecture qui va suivre de résultats d'essais pharmacologiques, mettant en oeuvre, le composé antiagrégant préféré suivant l'invention, à savoir l'acide 5-méthyl-5,6,7,8-tétrahydrofolique, en présence d'une substance connue pour ses propriétés

agrégantes, le méthotréxate, d'une part, et en présence d'un agent contraceptif, l'éthynyl-estradiol, d'autre part.

PREMIERE SERIE D'EXPERIENCES

Dans une première série d'expériences, les essais pharmacologiques ont été entrepris chez le rat mâle adulte, les rats étant répartis en lots de dix animaux chacun, à savoir :
un lot contrôle C recevant uniquement le véhicule, constitué de diméthylsulfoxyde et de NaCl à 9 g/l, par voie sous cutanée;
un lot traité M recevant le méthotréxate (0,5 mg/kg) dans le véhicule précité par voie sous-cutanée; et,
un lot traité N recevant le méthotréxate (0,5 mg/kg) dans le véhicule précité par voie sous-cutanée et l'acide 5-méthyl-5,6,7,8-tétrahydrofolique (0,5 mg/kg) également par voie sous-cutanée.

Les injections sont effectuées à heure fixe pendant 3 jours. Le quatrième jour, des prises de sang sont effectuées sur les animaux à jeun et les plaquettes sanguines sont isolées par les techniques habituelles et remises en suspension dans un tampon. L'activité agrégante de ces plaquettes est testée lors de la stimulation par des doses faibles de thrombine. Les résultats sont exprimés (en mm) par la pente de la courbe d'agrégation obtenue. Ces résultats sont consignés dans le tableau I ci-après.

**TABLEAU I**

| LOT | AGREGATION PLAQUETTAIRE (mm) | (%) |
|---|---|---|
| C | 20,4 ± 6,5 | 100 |
| M | 171,5 ± 9,7 | 855 |
| N | 8,7 ± 1,8 | 44 |

**DEUXIEME SERIE D'EXPERIENCES**

Dans une seconde série d'expériences, on a administré à des rats femelles pesant 200 à 300 g du méthotréxate par voie sous-cutanée ou un moyen contraceptif par intubation, d'une part, et l'acide folinique ou l'acide 5-méthyl-5,6,7,8-tétrahydrofolique, par voie sous-cutanée ou par gavage, d'autre part.

a) Des lots de rats femelles reçoivent du méthotréxate (0,5 mg/kg) par voie S.C. pendant trois jours. D'autres lots sont traités pendant la même durée par de l'acide folinique ou de l'acide 5-méthyl-5,6,7,8-tétrahydrofolique (0,5 mg/kg) par voie S.C. ou par voie orale (par gavage), le lot témoin recevant l'excipient du méthotréxate, l'excipient de l'acide folinique et l'excipient de l'acide 5-méthyl-5,6,7,8-tétrahydrofolique. Les analyses sont effectuées le lendemain du dernier jour de traitement sur les animaux à jeun. Les prises de sang ont lieu par la veine jugulaire après anesthésie légère. Les prélèvements sont effectués pour préparation des plasmas, des hématies et des plaquettes sanguines.

Agrégation sur plasma riche en plaquettes (PRP)

L'induction de l'agrégation est effectuée par la thrombine de l'ADP. Le méthrothréxate est administré entre 9 et 10 heures les jours $J_1$, $J_2$ et $J_3$, l'acide folinique et l'acide 5-méthyl-5,6,7,8-tétrahydrofolique sont administrés entre 9 et 10 heures les jours $J_1$, $J_2$ et $J_3$. Les prélèvements ont lieu le jour $J_4$ sur les animaux à jeun.

Les résultats obtenus figurent dans le tableau II ci-après.

Agrégation de plaquettes isolées

L'induction de l'agrégation est effectuée par la thrombine de l'ADP. Le méthrotréxate est administré entre 9 et 10 heures les jours $J_1$, $J_2$ et $J_3$, l'acide folinique et l'acide 5-méthyl-5,6,7,8-tétrahydrofolique sont administrés entre 9 et 10 heures les jours $J_1$, $J_2$ et $J_3$. Les prélèvements ont lieu le jour $J_4$ sur les animaux à jeun.

Les résultats obtenus figurent dans le tableau III ci-après.

b)Des lots de rats femelles reçoivent un moyen contraceptif : 5 microgrammes d'éthynyl-estradiol et 250 microgrammes de lynestrénol par voie orale (intubation) pendant quatre jours. D'autres lots sont traités pendant la même durée par de l'acide folinique ou de l'acide 5-méthyl-5,6,7,8-tétrahydrofolique (0,5 mg/kg) par voie S.C. ou par voie orale (par gavage), le lot témoin recevant l'excipient du moyen contraceptif , l'excipient de l'acide folinique et l'excipient de l'acide 5-méthyl-5,6,7,8-tétrahydrofolique. Les analyses sont effectuées le lendemain du dernier jour de traitement sur les animaux à jeun. Les prises de sang ont lieu par la veine jugulaire après anesthésie légère. Les prélèvements sont effectués pour préparation des plasmas, des hématies et des plaquettes sanguines.

Agrégation sur plasma riche en plaquettes (RPR)

L'induction de l'agrégation est effectuée par la thrombine de l'ADP. Le moyen contraceptif est administré entre 9 et 10 heures les jours $J_1$, $J_2$, $J_3$ et $J_4$ l'acide folinique et l'acide 5-méthyl-5,6,7,8-tétrahydrofolinique sont administrés entre 9 et 10 heures les jours $J_1$, $J_2$, $J_3$ et $J_4$. Les prélèvements ont lieu le jour $J_5$ sur les animaux à jeun.

Les résultats obtenus figurent dans le tableau IV ci-après.

Agrégation de plaquettes isolées

L'induction de l'agrégation est effectuée par la thrombine et l'ADP. Le moyen contraceptif est administré entre 9 et 10 heures les jours $J_1$, $J_2$, $J_3$ et $J_4$, l'acide folinique et l'acide 5-méthyl-5,6,7,8-tétrahydrofolique sont administrés entre 9 et 10 heures les jours $J_1$, $J_2$, $J_3$ et $J_4$. Les prélèvements ont lieu le jour $J_5$ sur les animaux à jeun.

Les résultats obtenus figurent dans le tableau V ci-après.

L'ensemble des résultats relatif aux mesures de l'agrégation plaquettaire est exprimé en millimètres sur les courbes d'agrégation suivant le système moyenne/écart type (n = 3 à 5). Ils mettent en évidence que le produit selon l'invention, à savoir l'acide 5-méthyl-5,6,7,8-tétrahydrofolique, désigné ici 5-N-Me-THF, manifeste surtout ses effets antiagrégants quand l'agrégation plaquettaire du rejet est trop importante.

TABLEAU II

| Produit | Agrégation plaquettaire (mm) | |
|---|---|---|
| | Thrombine | ADP |
| Contrôle | 55 ± 6 (d) | 132 ± 14 (d) |
| Acide folinique | 74 ± 11 | 136 ± 27 |
| 5-N-Me-THF | 65 ± 10 | 128 ± 9 |
| Méthotréxate | 135 ± 9 (d) | 202 ± 11 (d) |
| Méthotréxate + acide folinique | 120 ± 11 (a) | 141 ± 9 (c) |
| Méthotréxate + 5-N-Me-THF | 120 ± 11 (b) | 115 ± 8 (d) |

Notes

5-N-Me-THF : acide 5-méthyl-5,6,7,8-tétrahydrofolique;
(a) $p<0,05$, statistiquement significatif selon le test de Student;
(b) $p<0,025$, statistiquement significatif selon le test de Student;
(c) $p<0,005$, statistiquement significatif selon le test de Student;
(d) $p<0,001$, statistiquement significatif selon le test de Student.

**TABLEAU III**

| Produit | Agrégation plaquettaire (mm) | |
|---|---|---|
| | Thrombine | ADP |
| Contrôle | 118 ± 8 (c) | 130 ± 11 (d) |
| Acide folinique | 120 ± 11 | 126 ± 13 |
| 5-N-Me-THF | 82 ± 7 (b) | 101 ± 8 (a) |
| Méthotréxate | 161 ± 6 (d) | 231 ± 7 (d) |
| Méthotréxate + acide folinique | 136 ± 17 | 202 ± 14 |
| Méthotréxate + 5-N-Me-THF | 63 ± 6 (d) | 96 ± 6 (d) |

Notes

5-N-Me-THF : acide 5-méthyl-5,6,7,8-tétrahydrofolique;

(a) $p<0,05$, statistiquement significatif selon le test de Student;

(b) $p<0,025$, statistiquement significatif selon le test de Student;

(c) $p<0,005$, statistiquement significatif selon le test de Student;

(d) $p<0,001$, statistiquement significatif selon le test de Student.

**TABLEAU IV**

| Produit | Agrégation plaquettaire (mm)<br>Thrombine |
|---|---|
| Contrôle | 103 ± 7 |
| Moyen contraceptif (C) | 128 ± 17 |
| Moyen contraceptif + acide folinique | 113 ± 9 |
| Moyen contraceptif + 5-N-Me-THF | 99 ± 11 |

Notes

5-N-Me-THF : acide 5-méthyl-5,6,7,8-tétrahydrofolique;
(C): éthynyl-estradiol et lynestrénol.

**TABLEAU V**

| Produit | Agrégation plaquettaire (mm) | |
|---|---|---|
| | Thrombine | ADP |
| Contrôle | 132 ± 9 | 171 ± 8 (d) |
| Acide folinique | 140 ± 18 | 124 ± 19 (a) |
| 5-N-Me-THF | 160 ± 31 | 112 ± 16 (b) |
| Moyen contraceptif (C) | 172 ± 24 (c) | 328 ± 22 (d) |
| Moyen contraceptif + acide folinique | 161 ± 18 | 238 ± 7 (c) |
| Moyen contraceptif + 5-N-Me-THF | 61 ± 8 (c) | 144 ± 12 (d) |

**Notes**

5-N-Me-THF : acide 5-méthyl-5,6,7,8-tétrahydrofolique;
(C): éthynyl-estradiol + lynestrénol;
(a) $p<0,05$, statistiquement significatif selon le test de Student;
(b) $p<0,025$, statistiquement significatif selon le test de Student;
(c) $p<0,005$, statistiquement significatif selon le test de Student;
(d) $p<0,001$, statistiquement significatif selon le test de Student.

## Revendications

1. Utilisation en thérapeutique humaine ou vétérinaire d'une substance folinique, ladite utilisation étant caractérisée en ce que l'on fait appel à une substance choisie parmi

(i) l'acide 5-méthyl-5,6,7,8-tétrahydrofolique,
(ii) ses sels avec les métaux alcalins et alcalino terreux, et
(iii) leurs mélanges,
pour l'obtention d'un médicament antiagrégant plaquettaire destiné à une indication thérapeutique dans les cas où il convient d'inhiber ou réduire chez le mammifère l'agrégation des plaquettes sanguines.

2. Utilisation suivant la revendication 1, pour inhiber ou réduire chez l'homme l'agrégation des plaquettes sanguines.

3. Utilisation suivant la revendication 1, caractérisée en ce que ladite substance antiagrégante plaquettaire est l'acide 5-méthyl-5,6,7,8-tétrahydrofolique ou l'un de ses sels de sodium, potassium et calcium.

## Patentansprüche

1. Verwendung eines Folinsäurederivates in der Human- oder Veterinär-Therapeutik, wobei die Verwendung dadurch gekennzeichnet ist, dass man eine Substanz verwendet, die ausgewählt ist aus
(i) 5-Methyl-5,6,7,8-tetrahydrofolinsäure,
(ii) ihren Salzen mit Alkali- und Erdalkalimetallen und
(iii) ihren Mischungen,
um ein die Plättchenaggregation hemmendes Medikament zu erhalten, das für eine therapeutische Indikation in den Fällen bestimmt ist, in denen es ratsam ist, die Aggregation der Blutplättchen beim Säuretier zu inhibieren oder zu reduzieren.

2. Verwendung gemäss Anspruch 1, um die Aggregation der Blutplättchen beim Menschen zu inhibieren oder zu reduzieren.

3. Verwendung gemäss Anspruch 1, dadurch gekennzeichnet, dass die Plättchenaggregation hemmende Substanz 5-Methyl-5,6,7,8-tetrahydrofolinsäure oder eines ihrer Natrium-, Kalium- und Calcium-Salze ist.

## Claims

1. A use in human and veterinary therapy of a folinic acid substance, said use being characterized in that a substance selected from :
(i) 5-methyl-5,6,7,8-tetrahydrofolic acid,
(ii) its salts with alkaline and alkalino-eath metals, and
(iii) mixtures thereof,
is used in order to obtain a platelet aggregation inhibiting drug intended for a therapeutic indication in cases where it is appropriate to inhibit or reduce blood platelet aggregation.

2. A use according to claim 1 for inhibiting or reducing blood platelet aggregation in human being.

3. A use according to claim 1, characterized in that said platelet aggregation inhibiting substance is 5-methyl-5,6,7,8-tetrahydrofolic acid or one of its sodium, potassium and calcium salts.